# FASCICULE DE BREVET EUROPEEN

(11) **EP 1 569 614 B1**
(45) Date de publication et mention de la délivrance du brevet: **22.08.2012**
(21) Numéro de dépôt: 03796135.6
(22) Date de dépôt: 02.12.2003
(51) Int. Cl.: A61Q 19/00, A61K 8/97, A61K 8/98

(54) **UTILISATION D'UN EXTRAIT DE MIELLAT DE COTON COMME INGREDIENT ACTIF**
VERWENDUNG EINES IM EXTRAKT EINER BAUMWOLLE ENTHALTENEN HONIGTAUS ALS WIRKSTOFF
USE OF A COTTON HONEYDEW EXTRACT AS ACTIVE INGREDIENT IN OR FOR PREPARING A COSMETIC AND/OR PHARMACEUTICAL COMPOSITION

(30) Priorité: 03.12.2002 FR 0215189
(43) Date de publication de la demande: 07.09.2005
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2003/003557
(87) Numéro de publication internationale: WO 2004/052331

(56) Documents cités:
- EP-A- 0 622 487
- DATABASE WPI Section Ch, Week 199916 Derwent Publications Ltd., London, GB; Class B04, AN 1999-186170 XP002256877 & JP 11 035428 A (SHISEIDO CO LTD) 9 février 1999 (1999-02-09)
- DATABASE WPI Section Ch, Week 199513 Derwent Publications Ltd., London, GB; Class A96, AN 1995-093730 XP002256878 & JP 07 017824 A (SANSEI SEIYAKU KK) 20 janvier 1995 (1995-01-20)
- HENDRIX D.L.; WEI Y.A.; LEGGETT J.E.: "Homopteran honeydew sugar composition is determined by both the insect and plant species", COMPARATIVEBIOCHEMISTRY, vol. 101, no. 1-2, 1 August 1976 (1976-08-01), pages 23-27, XP022915235, PERGAMON PRESS, LONDON, GB

## Description

L'invention concerne le domaine de la cosmétique ainsi que le domaine de la pharmaceutique, notamment le domaine de la dermatologie.

La présente invention a pour objet l'utilisation, à titre de principe actif, dans ou pour la préparation d'une composition, d'une quantité efficace d'au moins un extrait de miellat de coton, l'extrait ou la composition étant destinés aux soins des substrats kératiniques.

La peau est le substrat kératinique majeur de l'organisme. C'est un organe vital recouvrant la totalité de la surface du corps et assurant des fonctions multiples telles que des fonctions sensitives, protectrices vis-à-vis d'agressions externes multiples, immunitaires, métaboliques ou encore thermorégulatrices. Ces rôles sont rendus possibles grâce à une structure complexe qui associe des structures tissulaires variées.

La peau est constituée de trois compartiments distincts superposés : l'épiderme, le derme et l'hypoderme. L'épiderme est un épithélium de revêtement qui constitue la structure externe de la peau et assure sa fonction de protection. Cette fonction est assurée par la cohésion des cellules épithéliales et par la production d'une protéine filamenteuse et résistante : la kératine. L'épiderme est caractérisé par une organisation en strate correspondant à un état de différenciation croissant des kératinocytes (cellule représentant plus de 80 % de la population cellulaire de l'épiderme) de la zone la plus profonde (*stratum basal*) à la zone la plus superficielle (*stratum corneum*). Pendant leur migration vers la surface, les kératinocytes subissent des modifications biochimiques et structurales dont la plus importante est la kératinisation, processus par lequel les cellules synthétisent la kératine. La couche cornée (*stratum corneum*) est ainsi très résistante aux agressions externes.

La kératine est la composante essentielle de tous les substrats kératiniques tels que les fibres capillaires, les poils, les ongles et autres phanères. La kératine est ainsi une molécule très importante au niveau du follicule pileux. Elle est produite par les kératinocytes situés au fond du bulbe pilaire qui se multiplient et se différencient. Certains se répartissent à la périphérie du follicule pileux pour former les gaines épithéliales externes et internes, et d'autres s'allongent pour former la tige pilaire. Au cours de ce trajet, les kératinocytes se chargent de fibres de kératine, ce qui rend le cheveu très résistant. La quantité de kératine dans la cellule joue donc un rôle très important, notamment dans tous les phénomènes de protection.

Les professionnels de la santé et de la cosmétique recherchent depuis de nombreuses années des moyens afin d'entretenir les substrats kératiniques, notamment la peau et les cheveux, ainsi que des moyens afin d'augmenter la résistance de la peau et des cheveux aux agressions extérieures et au stress qu'ils subissent quotidiennement. Un certain nombre de substances introduites dans des produits cosmétiques ou pharmaceutiques ont vu le jour, mais il reste encore des progrès à faire afin de pouvoir disposer de produits cosmétiques ou pharmaceutiques capables de régler ces problèmes de manière satisfaisante.

Le problème technique à résoudre a donc été, pour les inventeurs, de trouver une nouvelle substance, cosmétiquement ou pharmaceutiquement acceptable, qui soit capable d'apporter de véritables soins aux substrats kératiniques mais aussi de protéger la peau et les cheveux d'une manière efficace afin qu'ils ne subissent pas les dégradations occasionnées par les agressions et les stress d'origine extérieure.

Les inventeurs ont réussi à sélectionner des substances particulières présentant des propriétés remarquables lorsque celles-ci sont appliquées sur un substrat kératinique. De manière inattendue, les inventeurs ont découvert qu'une quantité efficace d'au moins un extrait de miellat de coton a des propriétés remarquables au niveau des substrats kératiniques et, notamment, qu'il protége la peau et les cheveux.

Le coton est l'ensemble des fibres (ou poils) recouvrant la graine du cotonnier. Le cotonnier, ou *Gossypium*, est une dicotylédone de la famille des malvacées. On compte de 40 à 50 espèces vivaces ou annuelles, ligneuses ou herbacées. Seules quatre d'entre elles sont cultivées pour leur fibre : *Gossypium hirsutum*, *Gossypium barbadense*, *Gossypium herbaceum* et *Gossypium arboreum.* Ces quatre espèces de cotonniers ont donné naissance à de nombreuses variétés hybrides, classées selon la longueur de leur fibre, la pubescence de la graine et la forme des bractées. C'est la lignée des cotonniers *Gossypium hirsutum*, qui fournit à lui seul de 80 à 90 % de la production mondiale de coton. Le fruit du cotonnier est une capsule ovale, coriace, et renfermant de très nombreuses graines portant des poils (ou fibres) serrés de longueurs variées.

Le coton occupe une place prépondérante parmi les fibres textiles naturelles telles que le chanvre, le lin et le jute. Depuis le XIXe siècle, il constitue la première fibre du monde grâce aux progrès des techniques industrielles et de la recherche agronomique. Ses linters, duvet subsistant sur les graines après l'égrenage, sont aussi très recherchés par les industries chimiques, et sa graine, contenant de 16 à 18 % d'huile comestible, intervient de plus en plus dans l'alimentation humaine et animale.

Le miellat désigne un produit, présent sur les plantes, contenant des sucres synthétisés par certains insectes ainsi que des sucres d'origine physiologique, c'est-à-dire synthétisés par la plante elle-même. Ce produit est, en partie, excrété par des insectes, comme, par exemple, les aleurodes et les pucerons (Gameel, 1996) lorsqu'ils se nourrissent de plantes et se dépose alors sur celles-ci. Le miellat se retrouve alors sur les plantes et crée une substance collante. Ce phénomène est particulièrement ennuyeux dans le cas du coton où la contamination par le miellat est l'un des facteurs les plus dommageables en terme de qualité des fibres de coton et pose de sérieux problèmes à l'industrie du textile.

A la connaissance de la demanderesse, il n'a jamais été décrit dans l'art antérieur l'utilisation d'extraits de miellat de coton dans des compositions cosmétiques et/ou pharmaceutiques et/ou dermatologiques. Jusqu'à présent seulement les huiles des graines de coton ainsi que des fibres de coton ont été utilisées en cosmétique, comme par exemple dans le brevet US 5,466,441 ou dans le brevet EP 1092425.

Ainsi, selon un premier aspect, la présente invention a pour objet l'utilisation cosmétique, à titre de principe actif, dans ou pour la préparation d'une composition, d'une quantité efficace d'au moins un extrait de miellat de coton, l'extrait ou la composition étant destinés aux soins des substrats kératiniques.

Le terme « extrait » désigne tout produit ou préparation obtenu à partir de substance végétale (ou animale) sèche. Il s'obtient, par exemple, par dissolution des actifs au moyen de solvants (comme l'eau, l'alcool ou encore l'éther) puis par concentration de ces actifs, par exemple, par évaporation des solvants. Par l'expression « extrait de miellat de coton », on entend toutes substances isolées, obtenues à partir de la matière première végétale que constitue le coton et, plus particulièrement, les fibres de coton. Cet extrait contiendra le maximum de composés extractibles. On entend, notamment, par l'expression « extrait de miellat de coton », tout extrait contenant des sucres récoltés à partir des fibres de coton. L'extrait de miellat de coton selon l'invention, obtenu par une méthode classique d'extraction bien connue de l'homme du métier, permettra d'obtenir une composition contenant différents types de molécules que l'on retrouve dans les fibres de cotons et, notamment, constitutif du miellat de coton. Ces molécules pourront être, plus spécialement, les différents types de sucres constitutifs de ce miellat. Bien entendu, l'extrait peut être préparé à partir du coton d'au moins l'une quelconque des nombreuses variétés et espèces de cotonniers.

Le principe actif peut se définir comme étant une molécule ou un ensemble de molécules susceptibles d'apporter des modifications ou des modulations au fonctionnement d'un système biologique.

Dans l'invention, on entend, par l'expression «substrat kératinique», tous les substrats qui sont composés en grande partie de kératine. Ce sont des substrats tels que la peau, les cheveux, les cils, les sourcils ou encore les ongles et les phanères en général.

Par les soins des substrats kératiniques, on entend toutes les actions destinées à conserver ou à rétablir un bon fonctionnement de ce substrat ou encore tout moyen qui sert à préserver ou à améliorer son apparence et/ou son aspect. Ainsi le soin inclus l'hydratation, l'apaisement, la protection contre tous types d'agression, notamment la protection solaire, la lutte et la prévention des manifestations du vieillissement, notamment les manifestations cutanées du vieillissement.

Par modifications cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutive à une exposition aux rayonnements ultra-violets.

Les cheveux et la peau sont sensibilisés et fragilisées à des degrés divers par l'action d'agents atmosphériques et par la lumière, ainsi que par des traitements plus ou moins agressifs tels que les permanentes, la teinture, les décolorations en ce qui concerne les cheveux, et les produits chimiques détergents, savons, produits de maquillage en, ce qui concerne la peau. Les propriétés mécaniques des cheveux et de la peau comme la résistance ou l'élasticité, sont, à la longue, altérées. Cette altération est due, en grande partie, à la détérioration plus ou moins importante de la kératine.

Pour le nettoyage et/ou le lavage des cheveux et/ou de la peau, l'utilisation de lotions détergentes (tels que des shampooings et autre savons) à base essentiellement d'agents tensioactifs classiques notamment de type anionique, non-anionique et/ou amphotère, mais plus particulièrement de type anionique, est courante. Ces compositions sont appliquées sur les substrats kératiniques mouillées et la mousse générée par le massage permet, après rinçage à l'eau, l'élimination des différentes salissures initialement présentes.

Ces compositions possèdent, certes, un bon pouvoir lavant mais les propriétés cosmétiques qui leurs sont attachées restent toutefois assez faibles compte tenu du caractère relativement agressif d'un tel traitement nettoyant. En effet, ce traitement peut, à la longue, occasionner sur la fibre capillaire et/ou sur la peau des dommages plus ou moins marqués, liés en particulier à une élimination progressive des protéines contenues dans ou à la surface de ces dernières. Ainsi, pour améliorer les propriétés cosmétiques des compositions détergeant ci-dessus, une solution est d'introduire des agents cosmétiques complémentaires destinés principalement à réparer ou à limiter les effets néfastes ou indésirables induits par les différents traitements ou agressions que subissent les fibres capillaires et la peau, c'est à dire à protéger. Ces agents cosmétiques pourront être, par exemple, des extraits de miellat de coton selon l'invention.

L'invention a pour second objet l'utilisation, cosmétique a titre de principe actif, dans ou pour la préparation d'une composition, d'une quantité efficace d'au moins un extrait de miellat de coton, l'extrait ou la composition étant destinés à protéger les substrats kératiniques, et plus particulièrement à protéger la peau et/ou les cheveux, contre tous les types d'agressions extérieures. L'extrait de miellat de coton selon l'invention, ou la composition le contenant, est avantageusement utilisé pour la protection des cheveux.

L'utilisation d'un extrait de miellat de coton va permettre aux substrats kératiniques d'être protégés et de mieux résister au stress que produit sur eux l'environnement. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums. Le types d'agression que subissent la peau et les cheveux sont dus, par exemple, à un déséquilibre du gradient électrochimique à travers la membrane cellulaire, ce qui peut aboutir à des variations importantes de la pression osmotique et cela peut avoir pour conséquence des chocs osmotiques et donc la lyse des cellules.

Or les inventeurs ont démontré, d'une manière surprenante, que l'extrait selon l'invention permet de protéger les cellules contre ces chocs osmotiques. De plus, il a été démontré que l'extrait selon l'invention permet d'obtenir une protection contre les dommages causés à l'ADN des cellules, notamment que l'extrait selon l'invention permet de protéger l'ADN des cellules lorsque celles-ci sont soumises à des stress tels que, par exemple, la privation de nutriments. Ces propriétés protectrices peuvent être utilisées, par exemple, pour réaliser des compositions destinées à protéger la peau et/ou les cheveux contre les agressions extérieures provoquées, notamment, par l'action du rayonnement solaire ou par d'autres agents physiques, chimiques ou biologiques. Ces propriétés protectrices peuvent aussi être utilisées pour réaliser des compositions permettant de lutter contre le vieillissement de la peau.

L'invention a, de plus, pour objet l'utilisation, à titre de principe actif, dans ou pour la préparation d'une composition, d'une quantité efficace d'au moins un extrait de miellat de coton, l'extrait ou la composition étant destinés à augmenter la synthèse des protéines des substrats kératiniques. En effet la demanderesse a démontré que l'extrait selon l'invention permet d'augmenter la synthèse des protéines des kératinocytes, notamment que l'extrait selon l'invention permet d'augmenter la synthèse de kératines.

Ainsi, selon une autre variante de l'invention, ledit agent actif augmente l'expression des protéines de la peau et/ou améliore leur stabilité. Cette protéine peut être représentative de l'état proliférant et/ou différentiant des cellules de la peau, plus particulièrement des cellules du derme et de l'épiderme. Notamment, la kératine est représentative de l'état proliférant et/ou différentiant des kératinocytes, plus précisément elle est représentative de l'état différentiant des kératinocytes. L'extrait selon l'invention a donc un rôle sur la différenciation cellulaire. L'extrait selon l'invention a donc une action positive sur la régénération tissulaire.

Ainsi, il a été démontré qu'un extrait de miellat de coton a une action renforçatrice sur la kératine du cheveu et de la peau, et plus généralement sur tous les substrats kératiniques, mais aussi qu'il possède un effet protecteur vis à vis de la lumière.

L'invention a aussi pour objet l'utilisation, à titre de principe actif, dans ou pour la préparation d'une composition, d'une quantité efficace d'au moins un extrait de miellat de coton, l'extrait ou la composition étant destinés à renforcer la barrière cutanée et/ou à renforcer la protection des cheveux. En outre, cet extrait de miellat de coton apporte d'excellentes propriétés cosmétiques aux cheveux et à la peau.

L'invention concerne aussi l'utilisation, à titre de principe actif, dans ou pour la préparation d'une composition, d'une quantité efficace d'au moins un extrait de miellat de coton, l'extrait ou la composition étant destinés à nourrir les substrats kératiniques. En effet, la demanderesse a démontré que l'extrait selon l'invention permet d'augmenter la survie des kératinocytes lorsque les cellules sont privées de nutriments. L'extrait selon l'invention permet aux cellules de mieux résister aux stress, notamment au stress occasionné par un manque de nutriments. L'extrait selon l'invention permet de renforcer la survie cellulaire. L'invention concerne aussi l'utilisation, à titre de principe actif, dans ou pour la préparation d'une composition, d'une quantité efficace d'au moins un extrait de miellat de coton, l'extrait ou la composition étant destinés à hydrater les substrats kératiniques.

Ainsi, l'extrait de miellat de coton , ou la composition le contenant, est particulièrement bien adapté aux soins des matières kératiniques et, tout particulièrement, aux soins des cheveux.

Le miellat de coton est constitué essentiellement de sucres. Il comprend, notamment, des sucres tels que le glucose, le fructose, le saccharose, le tréhalose, le tréhalulose ou encore le mélézitose. Le miellat de coton peut aussi contenir de l'inositol. Selon un mode de réalisation avantageux de l'invention, le miellat de coton possède des sucres présents dans des proportions définies. Ainsi, préférentiellement, le fructose peut représenter de 30 à 40 % de la quantité totale des sucres présents dans le miellat, le glucose peut représenter de 20 à 35 %, le saccharose de 3 à 20 %, le mélézitose de 0 à 10 %, le tréhalulose de 0 à 6 %, le tréhalose de 0 à 10 % et l'inositol de 0 à 12 % de la quantité totale des sucres présents dans le miellat.

Toute méthode d'extraction du miellat de coton peut être utilisée pour préparer l'extrait selon l'invention.

Ainsi, par exemple, le procédé d'extraction du miellat de coton peut se faire en plusieurs étapes. Dans une première étape, le miellat présent sur les fibres est extrait du coton avec l'aide d'un solvant. Ce solvant peut être d'origines diverses, il peut être composé d'eau et/ou de solvant organique (méthanol, éthanol, isopropanol, acétone...) et/ou de toute autre substance ayant la propriété de dissoudre les sucres.

Préférentiellement l'extraction est réalisée à l'aide d'un mélange eau-éthanol, la proportion d'alcool variant de 0 à 100 %. L'extraction peut être réalisée à différentes températures, à température ambiante ou bien à température supérieure, la température peut ainsi atteindre les 100°C.

Dans une deuxième étape, selon une variante du procédé d'extraction, un complexe enzymatique, favorisant l'extraction, peut être ajouté. Dans une autre étape, après extraction de l'extrait de miellat de coton, l'extrait peut être concentré par évaporation à pression atmosphérique ou sous vide. Cet extrait est alors repris dans un solvant approprié, puis stérilisé. L'extrait peut alors être utilisé en l'état.

Dans une variante de l'invention, l'extrait de miellat de coton peut subir un traitement d'hydrolyse par voie chimique ou par mise en contact avec un micro-organisme ou avec un complexe enzymatique. L'extrait peut aussi subir divers traitements de décoloration par contact avec des substances absorbantes, du charbon actif ou encore des terres décolorantes. L'extrait peut, de la même manière, subir des traitements de purification et de fractionnement par la technique de cristallisation ou grâce à un procédé de type chromatographique.

L'extrait selon l'invention peut être de différentes natures ; on peut en particulier citer les extraits aqueux, alcooliques ou utilisant un solvant organique. Par solvant aqueux on entend tout solvant constitué totalement ou pour part d'eau. On peut citer l'eau elle-même, les solvants hydroalcooliques en toute proportion ou encore les solvants constitués d'eau et d'un composé comme le propylène glycol ou le butylène glycol en toute proportion. Parmi les solvants alcooliques on peut citer, notamment, l'éthanol. Cet extrait peut être obtenu par dissolution dans l'eau, l'alcool ou l'éther, puis par concentration de cette solution en faisant intervenir l'évaporation ou la distillation.

Pour donner un ordre de grandeur, on peut utiliser l'extrait selon l'invention en une quantité représentant de 0,0001 % à 20 % du poids total de la composition et préférentiellement en une quantité représentant de 0,01 % à 10 % du poids total de la composition.

Selon un mode de réalisation avantageux de l'invention, l'extrait précité est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, l'extrait précité est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans ou fixés sur, tout vecteur cosmétiquement ou pharmaceutiquement acceptable.

Selon un autre aspect, l'invention a pour objet une composition selon la revendication 11 caractérisée en ce qu'elle contient, comme principe actif, dans un milieu cosmétiquement ou pharmaceutiquement acceptable, au moins un extrait de miellat de coton tel que défini précédemment.

La composition selon l'invention peut être une composition cosmétique et/ou dermatologique et/ou pharmaceutique. Préférentiellement selon l'invention, la composition est une composition cosmétique, car elle est destinée à améliorer l'aspect et les performances cutanées générales de l'individu qui en fait usage.

La composition selon l'invention est préférentiellement une composition cosmétique et/ou dermatologique adaptée à l'administration par voie topique cutanée comprenant un milieu cosmétiquement ou dermatologiquement acceptable.

Selon un mode de réalisation préféré, la composition est tout particulièrement bien adaptée à une utilisation pour le soin des cheveux.

Il est bien évident que l'invention s'adresse aux mammifères en général et plus particulièrement aux êtres humains.

La quantité efficace de principe actif correspond à la quantité nécessaire pour obtenir le résultat désiré. L'extrait de miellat de coton précité est présent dans les compositions de l'invention à une concentration comprise entre 0,0001 % à 20 % environ, et préférentiellement à une concentration comprise entre 0,01 % et 10 % environ en poids par rapport au poids total de la composition finale.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées.

Préférentiellement, les compositions selon la présente invention se présenteront sous une forme galénique adaptée à l'administration par voie topique cutanée et adaptée à une administration sur les cheveux. Elles couvrent toutes les formes cosmétiques ou dermatologiques. Ces compositions doivent donc contenir un milieu cosmétiquement acceptable, c'est-à-dire compatible avec la peau, les poils ou les cheveux. Ces compositions peuvent notamment se présenter sous forme d'une solution aqueuse, hydralcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau. Pour l'injection, la composition selon l'invention peut se présenter sous forme de lotion aqueuse, huileuse ou sous forme de sérum. Pour l'application sur les yeux, la composition peut se présenter sous forme de gouttes et pour l'ingestion, elle peut se présenter sous forme de capsules, de granulés, de sirops ou de comprimés.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs et des polymères filmogènes .

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas ou sensiblement pas, altérées par l'adjonction envisagée.

Les compositions selon l'invention trouvent une application notamment comme composition cosmétique ou pharmaceutique pour la peau, les muqueuses et/ou les semimuqueuses, mais aussi comme composition cosmétique pour les cheveux et/ou les poils. Elles trouvent une application toute particulière en tant que produit de protection et/ou de soins pour les cheveux.

On peut également envisager une application dans le domaine des compositions de maquillage de la peau du visage et du corps, telles que les rouges à lèvres, les fond de teint, les crèmes teintées, les sticks anti-cernes, les compositions anti-solaires ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, par exemple une pâte de dentifrice. La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule ou encore d'un aliment ou complément nutritionnel.

Selon l'invention, on peut, entre autres, ajouter à la composition de l'invention d'autres agents actifs destinés notamment à la prévention et/ou au traitement des manifestations cutanées du vieillissement et/ou à la protection de la peau et/ou des cheveux contre les agressions extérieures. La composition selon l'invention peut être destinée à former un filtre protecteur au niveau des substrats kératiniques. L'utilisation de l'extrait et de la composition selon l'invention peut être envisagée de manière curative et/ou préventive.

Selon un autre aspect, la présente invention concerne un procédé de traitement cosmétique pour les soins des substrats kératiniques et plus particulièrement pour le soin de la peau et/ou des cheveux consistant à appliquer sur la surface de la peau et/ou des cheveux une quantité efficace d'un extrait de miellat de coton, tel que défini précédemment, afin d'obtenir l'action désirée. La présente invention concerne, de la même manière, un procédé de traitement cosmétique afin de protéger les substrats kératiniques contre tous types d'agressions extérieures.

Des modes de réalisation particuliers de ce procédé de traitement cosmétique résultent également de la description précédente.

Selon un autre aspect de l'invention, la présente invention concerne un procédé de traitement cosmétique afin de renforcer la barrière cutanée de la peau et/ou de renforcer la protection des cheveux. Selon un autre aspect de l'invention, la présente invention concerne un procédé de traitement cosmétique afin d'augmenter la synthèse de kératine et/ou afin de nourrir la peau. Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

Selon un mode de réalisation particulier de l'invention, l'extrait de miellat de coton peut être utilisé pour la préparation d'une composition détergente ou pour la préparation de lessives. En effet, l'extrait de miellat de coton selon l'invention a une efficacité remarquable pour l'entretien et la protection des fibres naturelles ou synthétiques. Ainsi, selon encore un autre aspect de l'invention, l'invention a pour objet l'utilisation, dans ou pour la préparation d'une composition, d'une quantité efficace d'au moins un extrait de miellat de coton, l'extrait ou la composition étant destinés à l'entretien et/ou à la protection des fibres naturelles ou synthétiques.

D'autres avantages et caractéristiques de l'invention apparaîtront mieux à la lecture des exemples donnés à titre illustratif et non limitatif.

### Exemple 1 : Préparation de l'extrait de miellat de coton.

Dans une première étape, le miellat est extrait des fibres de coton à l'aide d'un solvant, ledit solvant étant constitué d'un mélange eau-éthanol, la proportion d'alcool variant de 0 à 100%.

Puis, dans une deuxième étape, les sucres sont extraits du coton par hydrolyse à l'aide d'un cocktail enzymatique, le pH de la réaction étant compris entre 4 et 8, la température étant comprise entre 35 et 65°C.

Les principaux sucres extraits sont le glucose, le fructose, le saccharose, le tréhalose, le mélézitose, le tréhalulose et l'inositol.

Le rendement d'extraction est de l'ordre de 0,3 %. La concentration finale en sucres est comprise entre 50 et 100 g/l.

Ces sucres sont ensuite présentés en solution aqueuse ou hydroalcoolique puis ils sont stérilisés par chauffage à 65 °C pendant 12 heures.

### Exemple 2 : Mise en évidence de l'effet de l'extrait de miellat de coton sur l'expression des kératines.

Le but de l'étude est de déterminer l'influence de l'extrait selon l'invention sur la synthèse de kératines, par les kératinocytes, à l'aide de la technique d'immunofluorescence, une technique semi-quantitative qui permet d'apprécier le taux de chacune des protéines présentes dans le cytoplasme cellulaire.

L'expression des kératines a été étudiée sur des coupes de peau humaine mises en culture pendant 24 heures. L'extrait de miellat de coton a été appliqué à 0,5 % (c'est-à-dire dilué dans le PBS à 0,5 %) sur les coupes de peau, à raison de deux applications. L'effet de l'extrait a été évalué par comparaison avec une coupe de peau non traitée avec l'extrait de miellat de coton selon l'invention. Après application, les échantillons de peau ont été cultivés pendant 24 heures, puis préparés pour l'inclusion dans la paraffine. L'immunomarquage a ensuite été réalisé à l'aide d'un anticorps anti-kératine. La détection de la quantité de kératine a été réalisée par l'immunofluorescence.

Les résultats obtenus démontrent que l'application de l'extrait selon l'invention, sur les coupes de peau, a pour effet d'augmenter la synthèse de kératine. Cette stimulation a été observée de manière importante par comparaison avec les coupes non traitées. En effet, lorsque les coupes de peau sont incubées en présence de la composition contenant les actifs, on assiste à une augmentation de l'intensité de la fluorescence traduisant ainsi une stimulation de la synthèse de kératine par les kératinocytes.

### Exemple 3 : Mise en évidence de l'effet nutritif de l'extrait de miellat de coton sur des kératinocytes.

Le but de l'étude est de démontrer l'effet nutritif de l'extrait selon l'invention sur les kératinocytes. Cet effet a été mesuré à l'aide d'un test de viabilité cellulaire, sur des cellules privées de nutriments.

L'étude a été réalisée sur des kératinocytes humains HaCaT, en phase de croissance exponentielle dans des Labtecks^{™}. Les kératinocytes ont été ensemencés dans des plaques de 96 puits. Des cellules sont prétraitées durant 2 heures avec l'actif. Puis le milieu de culture (contenant ou ne contenant pas l'actif) est remplacé par du PBS, en présence ou en l'absence d'actif, pendant une période de 3h30. La condition contrôle réalisée est constituée de cellules maintenues durant 3h30 dans du PBS (sans actif).

Des tests de viabilité cellulaire ont été réalisés grâce au test MTT. D'une manière générale, l'agent MTT est utilisé pour évaluer la cytotoxicité d'un produit vis-à-vis d'un milieu cellulaire par mesure de la viabilité cellulaire.

Les kératinocytes sont incubés dans une solution contenant 0,1 mg/ml de MTT (3-[4, 5-diméthylthiazol-2-yl]-2, 5-diphényltétrazolium bromide). Ce composé est absorbé par les cellules vivantes puis métabolisé par les enzymes mitochondriales (succinate déshydrogénase) en un composé bleu violet, le formazan, qui se présente sous forme de cristaux violets insolubles en milieu aqueux. Les cristaux de formazan sont solubilisés dans du DMSO, ils donnent une densité optique (D.O.) proportionnelle au nombre de cellules vivantes présentes dans l'échantillon. Des mesures de densité optique ont ensuite été réalisées pour chaque échantillon étudié (La D.O. se lisant à 540 nm). La D.O. est alors directement proportionnelle à l'activité enzymatique ainsi qu'au nombre de cellules vivantes.

Les résultats ont démontré que les cellules cultivées dans du PBS, c'est-à-dire totalement privées de nutriments, subissent un stress très intense et s'altèrent rapidement. De la même manière, ces mesures ont démontré que, selon les concentrations d'extrait utilisées, les cellules traitées avec ledit extrait ont une viabilité qui augmente de 15 % à 30 % par rapport aux cellules non traitées. Ces résultats démontrent ainsi clairement que l'extrait de miellat de coton selon l'invention possède un pouvoir nutritif important au niveau cellulaire.

### Exemple 4 : Mise en évidence de l'effet protecteur de l'extrait de miellat de coton contre le choc osmotique.

Le but de l'étude est de démontrer l'effet protecteur de l'extrait de miellat de coton contre le choc osmotique, sur les kératinocytes. Cet effet a été mesuré à l'aide d'un test de viabilité cellulaire, sur des cellules ayant subit un choc osmotique.

L'étude a été réalisée sur des kératinocytes humains HaCat, en phase de croissance exponentielle dans des Labtecks^{™}. Les cellules ont ensuite été traitées pendant 1 heure avec 0,5 % de l'extrait de miellat de coton. Puis le milieu a été retiré et les kératinocytes ont été incubés pendant 1 heure dans un milieu hypertonique (c'est-à-dire contenant du DMEM et 500 mM de NaCl), en présence ou en l'absence d'actif. Puis un test MTT (tel que décrit ci-dessus) a été réalisé afin d'étudier la viabilité cellulaire.

Le résultat de ce test démontre que la viabilité des cellules est diminuée de moitié lorsque celles-ci sont soumises à un choc osmotique. En outre, les résultats démontrent que le prétraitement avec l'extrait de miellat de coton augmente de 37 % la viabilité de kératinocytes par rapport aux cellules témoins, c'est-à-dire non traitées avec l'actif mais ayant subi le choc osmotique.

### Exemple 5 : Mise en évidence de l'effet de l'extrait de miellat de coton sur la protection de l'ADN.

Le but de l'étude est de démontrer l'effet protecteur de l'ADN, de l'extrait selon l'invention, sur les kératinocytes. Cet effet a été mesuré à l'aide d'un « test des Comètes » sur des cellules ayant subit un stress important.

Le même test que celui de l'exemple 3 a été effectué : les cellules ont été soumises à un stress provoqué par une privation de nutriments ("food starvation"). Suite à ce stress, une étude de la protection de l'ADN a été effectuée, grâce au test des Comètes, afin d'évaluer la dégradation de l'ADN des cellules traitées avec l'extrait de miellat de coton par comparaison avec les cellules non traitées avec l'extrait.

Le test des Comètes ou "Single Cell Gel Electrophoresis" est une technique microélectronique courte et sensible qui permet de visualiser les cassures de l'ADN simple et double-brin sur des cellules individuelles. Après traitement, les cellules sont emprisonnées dans un gel d'agarose et lysées dans un tampon à forte salinité contenant des détergents. L'ADN est ensuite dénaturé par bain alcalin suivi d'une courte électrophorèse, puis il est mis en évidence par l'iodure de propidium. L'ADN d'une cellule altérée s'étire vers l'anode proportionnellement au nombre de cassures et forme une comète. L'ADN fortement dégradé se retrouve dans la "queue" de la comète. Une cellule intacte reste ronde, l'ADN restant compacté au niveau de la "tête" de la comète. L'évaluation des lésions de l'ADN s'effectue à l'aide d'un logiciel qui permet de déterminer le pourcentage de la dégradation d'ADN.

Les résultats démontrent que les cellules soumises au "food starvation" subissent un stress et que leur ADN est dégradé. En outre, les résultats démontrent que la protection de l'ADN des cellules traitées avec l'extrait de miellat de coton est augmentée de 60 % par rapport aux cellules contrôles (c'est-à-dire les cellules non traitées par l'actif mais ayant subi le stress). L'extrait de miellat de coton joue donc un rôle important sur la protection de l'ADN.

### Exemple 6 - Préparation de compositions

Ces compositions ont été obtenues par simple mélange des différents composants. Les quantités indiquées sont données en pourcentage de poids.

### 1. Emulsion huile-dans-eau

| ***Noms commerciaux*** | ***Noms INCI*** | ***% massique*** |
|---|---|---|
| ***PHASE HUILEUSE*** | | |
| Montanov 68 | Cetearyl Alcohol (and) Cetearyl Glucoside | 5.00 |
| Huile de Jojoba | Simmondsia Chimensis Seed Oil | 5.00 |
| Huile de Vaseline | Paraffinum Liquidum (Minéral oil) | 5.00 |
| Isopropyl Palmitate | Isopropyl Palmitate | 7.00 |

| ***PHASE AQUEUSE*** | | |
|---|---|---|
| Glycerine | Glycerin | 5.00 |
| Allantoïne | Allantoin | 0.10 |
| Sepigel 305 | Polyacrylamide (and) C13-14 Isoparaffin (and) Laureth-7 | 0,30 |
| Extrait selon l'exemple 1 | | 1.00 |
| Conservateur | | 0,50 |
| Parfum | Parfum (Fragrance) | 0,50 |
| Eau déminéralisée | Aqua (Water) | Qsp |

### 2. Lotion

| ***Noms commerciaux*** | ***Noms INCI*** | ***% massique*** |
|---|---|---|
| Mono Propylene Glycol | Propylene Glycol | 1.00 |
| Allantoine | Allantoin | 0.30 |
| Glycérine | Glycerin | 1.00 |
| Cetiol HE | PEG-7 Glyceryl Cocoate | 1.00 |
| Extrait de l'exemple 1 | | 0.01 |
| Conservateur | | 0.20 |
| Parfum | Parfum (Fragrance) | 0.50 |
| Eau déminéralisée | Aqua (Water) | qsp |

### 3. Gel

| ***Noms commerciaux*** | ***Noms INCI*** | ***% mastique*** |
|---|---|---|
| Carbopol Ultrez 10 (2%) | Carbomer | 25.00 |
| Triéthanolamine | Triethanolamine | 0.50 |
| Extrait de l'exemple 1 | | 0.50 |
| Conservateur | | 0.20 |
| EDTA | Tetrasodium EDTA | 0.10 |
| Parfum | Parfum (Fragrance) | 0.50 |
| Colorant hydrosoluble | | Qsp |
| Eau déminéralisée | Aqua (Water) | Qsp |

### 4. Shampooing

| ***Noms commerciaux*** | ***Noms INCI*** | ***% massique*** |
|---|---|---|
| Texapon NSO | Sodium Laureth Sulfate | 30.00 |
| Tegobetaïne HS | Cocamidopropyl Betaïne | 6.00 |
| Tween 20 | Polysorbate 20 | 2.00 |
| Glucamate DOE 120 (sol à 50 %) | PEG-120 Methyl Glucose Dioleate | 0.75 |
| EDTA | Tetrasodium EDTA | 0.10 |
| Chlorure de Sodium | Sodium Chloride | 1.00 |
| Extrait selon l'exemple 1 | | 1.00 |
| Conservateur | | 0.30 |
| Parfum | Parfum (Fragrance) | 0.50 |
| Colorant hydrosoluble | | Qsp |
| Eau déminéralisée | Aqua (Water) | Qsp |
| Acide Citrique | Citric Acid | qsp pH= 5.5- 6.0 |

## Revendications

1. Utilisation cosmétique, à titre de principe actif d'une quantité efficace d'au moins un extrait de miellat de coton, l'extrait ou une composition contenant le dit extrait étant destinés aux soins des substrats kératiniques,

2. Utilisation selon la revendication 1, l'extrait ou la composition étant destinés à protéger les substrats kératiniques, et plus particulièrement à protéger la peau et/ou les cheveux contre tous types d'agressions extérieures.

3. Utilisation selon la revendication 1, l'extrait ou la composition étant destinés à augmenter la synthèse des protéine des substrats kératiniques, notamment des kératines.

4. Utilisation selon la revendication 1, l'extrait ou la compostion étant destinés à renforcer la barrière cutanée de la peau et/ou à renforcer la protection des cheveux.

5. Utilisation selon la revendication 1, l'extrait ou la composition étant destinés à nourrir les substrats kératiniques.

6. Utilisation selon la revendication 1, l'extrait ou la composition étant destinés à l'entretien et/ou à la protection des fibres naturelles ou synthétiques.

7. Utilisation selon l'une quelconque des revendications précédentes **caractérisée en ce que** l'extrait de miellat de coton comprend des sucres tels que le glucose, le fructose, le saccharose, le tréhalose, le tréhalulose, le mélézitose ou encore l'inositol.

8. Utilisation selon l'une quelconque des revendications précédentes **caractérisée en ce que** les sucres de l'extrait de miellat de coton sont présents en des proportions telles que le fructose représente de 30 à 40 % de la quantité totale des sucres présents dans le miellat, le glucose représente de 20 à 35 %, le saccharose de 3 à 20 %, le mélézitose de 0 à 10 %, le tréhalulose de 0 à 6 %, le trehalose de 0 à 10 % et l'inositol de 0 à 12 % de la quantité totale des sucres présents dans le miellat.

9. Utilisation selon l'une quelconque des revendications précédentes **caractérisée en ce que** l'extrait de miellat de coton est utilisé dans des proportions comprises entre 0,0001 % et 20 % en poids par rapport au poids total de la composition, préférentiellement dans des proportions comprises entre 0,01 % et 10 % en poids par rapport au poids total de la composition.

10. Utilisation selon l'une quelconque des revendications précédentes **caractérisée en ce que** l'extrait de miellat de coton est un extrait de miellat de fibres de coton.

11. Composition cosmétique et/ou dermatologique et/ou pharmaceutique **caractérisée en ce qu'**elle contient comme principe actif, dans un milieu cosmétiquement, pharmaceutiquement ou dermatologiquement acceptable, au moins un extrait de miellat de coton, l'extrait de miellat de coton étant présent dans la composition à une concentration comprise entre 0,0001 % et 20 % en poids par rapport au poids total de la composition, et préférentiellement à une concentration comprise entre 0,001 % et 10 % en poids par rapport au poids total de la composition.

12. Composition selon la revendication 11, **caractérisée en ce qu'**elle se présente sous la forme d'une composition cosmétique et/ou dermatologique adaptée à l'administration par voie topique cutanée comprenant un milieu cosmétiquement ou dermatologiquement acceptable.

13. Composition selon l'une quelconque des revendications 11 ou 12 **caractérisée en ce que** l'extrait est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, la vaseline, une huile végétale ou tout mélange de ces solvants.

14. Composition selon l'une quelconque des revendications 11 ou 12 **caractérisée en ce que** l'extrait est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

15. Composition selon l'une quelconque des revendications 11 ou 12, **caractérisée en ce qu'**elle se présente sous forme d'une solution aqueuse, hydralcoolique ou huileuse ou sous la forme d'une émulsion huile-dans-eau, eau-dans-huile ou émulsions multiples ou sous forme de crèmes, de suspensions, ou encore de poudres ; ces compositions pouvant être plus ou moins fluides ou solides et avoir l'aspect d'une crème, d'une lotion, d'un lait, d'un shampooing, d'un sérum, d'une pommade, d'un gel, d'une pâte, d'une mousse ou d'un stick.

16. Procédé de traitement cosmétique pour les soins des substrats kératiniques, et plus particulièrement de la peau et des cheveux, consistant à appliquer sur les substrats kératiniques, et plus particulièrement sur la peau et/ou les cheveux, une quantité efficace d'une composition telle que définie dans l'une quelconque des revendications 11 à 15.

17. Procédé de traitement cosmétique selon la revendication 16 pour protéger les substrats kératiniques, et plus particulièrement pour protéger la peau et les cheveux, contre tous les types d'agressions extérieures.

18. Procédé de traitement cosmétique selon la revendication 16 afin de renforcer la barrière cutanée de la peau et/ou afin de renforcer la protection des cheveux, et/ou afin d'augmenter la synthèse de kératine et/ou afin de nourrir les substrats kératiniques, et plus particulièrement la peau et les cheveux.

## Claims

1. Use, as active ingredient, of an effective amount of at least one cotton honeydew extract, the extract or a composition containing said extract being intended for the care of keratin substrates.

2. The use according to claim 1, wherein the extract or composition is intended to protect keratin substrates, in particular to protect the skin and/or hair against all types of external aggressions.

3. The use according to claim 1, wherein the extract or composition is intended to increase the synthesis of proteins of keratin substrates, in particular keratins.

4. The use according to claim 1, wherein the extract or composition is intended to strengthen the cutaneous barrier of the skin and/or strengthen hair protection.

5. The use according to claim 1, wherein the extract or composition is intended to nourish the keratin substrates.

6. The use according to claim 1, wherein the extract or composition is intended for the care and/or protection of natural or synthetic fibers.

7. The use according to any one of the preceding claims, **characterized in that** the cotton honeydew extract comprises sugars such as glucose, fructose, sucrose, trehalose, trehalulose, melezitose or inositol.

8. The use according to any one of the preceding claims, **characterized in that** the sugars in the cotton honeydew extract are present in proportions such that fructose is in the range from 30 to 40% of the total amount of sugars in the honeydew, glucose is in the range from 20 to 35%, sucrose, from 3 to 20%, melezitose, from 0 to 10%, trehalulose, from 0 to 6%, trehalose, from 0 to 10% and inositol, from 0 to 12% of the total amount of sugars in the honeydew.

9. The use according to any one of the preceding claims, **characterized in that** the cotton honeydew extract is used in proportions of between 0.0001% and 20% by weight relative to the total weight of the composition, preferably in proportions of between 0.01% and 10% by weight relative to the total weight of the composition.

10. The use according to any one of the preceding claims, **characterized in that** the cotton honeydew extract is a honeydew extract of cotton fibers.

11. A cosmetic and/or dermatological and/or pharmaceutical composition, **characterized in that** it contains, as active ingredient, in a cosmetically, pharmaceutically or dermatologically acceptable medium, at least one cotton honeydew extract, the cotton honeydew extract being present at a concentration of between 0.0001% and 20% by weight relative to the total weight of the composition, preferably at a concentration of between 0.001% and 10% by weight relative to the total weight of the composition.

12. The composition according to claim 11, **characterized in that** it is in the form of a cosmetic and/or dermatological composition adapted for topical administration on the skin, comprising a cosmetically or dermatologically acceptable medium.

13. The composition according to any one of claims 11 or 12 **characterized in that** the extract is previously solubilized in one or more cosmetically or pharmaceutically acceptable solvents such as water, ethanol, propanol or isopropanol, propylene glycol, butylene glycol, dipropylene glycol, ethoxylated or propoxylated diglycols, cyclic polyols, petroleum jelly, a vegetal oil or any mixture of these solvents.

14. The composition according to any one of claims 11 or 12, **characterized in that** the extract is previously solubilized in a cosmetic or pharmaceutical vector such as liposomes or adsorbed onto powdery organic polymers, mineral supports such as talcs and bentonites, and more generally dissolved in, or fixed to, any cosmetically or pharmaceutically acceptable vector.

15. The composition according to any one of claims 11 or 12, **characterized in that** it is in the form of an aqueous, hydro-alcoholic or oily solution or in the form of an oil-in-water, water-in-oil emulsion or of multiple emulsions or in the form of creams, suspensions, or powders; wherein these compositions can be more or less fluid or solid and have the appearance of a cream, a lotion, a milk, a shampoo, a serum, an ointment, a gel, a paste, a mousse or a stick.

16. A cosmetic treatment process for the care of keratin substrates, in particular the skin and hair, comprising applying an effective amount of a composition such as defined in any one of claims 11 to 15, onto keratin substrates, in particular to the skin and/or hair.

17. The cosmetic treatment process of claim 16 for protecting keratin substrates, in particular for protecting the skin and hair against all types of external aggressions.

18. The cosmetic treatment process of claim 16, for strengthening of the cutaneous barrier of the skin and/or strengthening protection of the hair, and/or increasing the synthesis of keratin and/or nourishing the keratin substrates, in particular the skin and hair.

## Patentansprüche

1. Kosmetische Anwendung, als Wirkstoff, einer wirksame Menge von zumindest einem Baumwollhonigtauextrakt, wobei der Extrakt oder eine Zusammensetzung, die das genannte Extrakt enthält, dazu bestimmt ist Keratinsubstrate zu pflegen.

2. Anwendung nach Anspruch 1, wobei der Extrakt oder die Zusammensetzung dazu bestimmt ist, die Keratinsubstrate zu schützen, und insbesondere, die Haut und/oder die Haare gegen alle Arten äußeren Angriffen zu schützen.

3. Anwendung nach Anspruch 1, wobei der Extrakt oder die Zusammensetzung dazu bestimmt ist, die Synthese von Proteinen der Keratinsubstrate, insbesondere Keratinen, zu erhöhen.

4. Anwendung nach Anspruch 1, wobei der Extrakt oder die Zusammensetzung dazu bestimmt ist, die Hautschranke zu verstärken und/oder den Schutz der Haare zu verstärken.

5. Anwendung nach Anspruch 1, wobei der Extrakt oder die Zusammensetzung dazu bestimmt ist, die Keratinsubstrate zu nähren.

6. Anwendung nach Anspruch 1, wobei der Extrakt oder die Zusammensetzung für die Pflege und/oder den Schutz der natürlichen oder synthetischen Fasern bestimmt ist.

7. Anwendung nach einem der vorhergehenden Ansprüche, **dadurch gekennzeichnet, dass** der Extrakt des Baumwollhonigtaus Zucker wie Glukose, Fruktose, Saccharose, Trehalose, Trehalulose, Melezitose oder auch Inositol enthält.

8. Anwendung nach einem der vorhergehenden Ansprüche, **dadurch gekennzeichnet, dass** die Zucker des Baumwollhonigtauextrakts in solchen Anteilen auftreten, dass die Fruktose 30 bis 40% der Gesamtmenge der im Honigtau vorhandenen Zucker ausmacht, Glukose zwischen 20 und 35%, Saccharose zwischen 3 und 20%, Melezitose zwischen 0 und 10 %, Trehalulose zwischen 0 und 6%, Trehalose zwischen 0 und 10 % und Inositol zwischen 0 und 12% ausmacht, der Gesamtmenge der im Honigtau vorhandenen Zucker.

9. Anwendung nach einem der vorhergehenden Ansprüche, **dadurch gekennzeichnet, dass** der Baumwollhonigtauextrakt in Gewichtsanteilen verwendet wird, die zwischen 0.0001% und 20% des Gesamtgewichts der Zusammensetzung vorliegen, vorzugsweise in Anteilen zwischen 0.01% und 10% bezogen auf das Gesamtgewicht der Zusammensetzung.

10. Anwendung nach einem der vorhergehenden Ansprüche, **dadurch gekennzeichnet, dass** der Baumwollhonigtauextrakt ein Honigtauextrakt aus Baumwollfasern ist.

11. Kosmetische und/oder dermatologische und/oder pharmazeutische Zusammensetzung, **dadurch gekennzeichnet, dass** diese als Wirkstoff in einem kosmetisch, pharmazeutisch oder dermatologisch akzeptables Medium, zumindest ein Baumwollhonigtauextrakt enthält, wobei der Baumwollhonigtauextrakt in der Zusammensetzung eine Konzentration aufweist, die zwischen 0.0001% und 20 Gew.% bezogen auf das Gesamtgewicht der Zusammensetzung, und vorzugsweise eine Konzentration aufweist, die zwischen 0.001% und 10 Gew.% bezogen auf das Gesamtgewicht der Zusammensetzung liegt.

12. Zusammensetzung nach Anspruch 11, **dadurch gekennzeichnet, dass** sie in Form einer kosmetischen und/oder dermatologischen Zusammensetzung ausgebildet ist, die zur topischen Verabreichung auf der Haut angepasst ist mit ein kosmetisches oder dermatologisch akzeptables Medium.

13. Zusammensetzung nach einem der Ansprüche 11 oder 12, **dadurch gekennzeichnet, dass** der Extrakt zuvor in einem oder mehreren kosmetisch oder pharmazeutisch akzeptablen Lösungsmittel, wie Wasser, Ethanol, Propanol oder Isopropanol, Propylenglykol, Butylenglykol, Dipropylenglykol, ethoxylierten oder propoxylierten Diglykolen, cyclische Polyolen, Vaseline, pflanzlichem Öl oder jegliche Mischung dieser Lösungsmittel solubilisiert wurde.

14. Zusammensetzung nach einem der Ansprüche 11 oder 12, **dadurch gekennzeichnet, dass** der Extrakt zuvor in einem kosmetischen oder pharmazeutischen Vektor, wie Liposomen solubilisiert wurde oder auf pulverförmigen organischen Polymeren, mineralischen Trägern wie Talken oder Bentoniten adsorbiert wurde, und noch genereller, in jeglichem kosmetisch oder pharmazeutisch akzeptabel Vektor solubilisiert, oder daran fixiert wurde.

15. Zusammensetzung nach einem der Ansprüche 11 oder 12, **dadurch gekennzeichnet, dass** sie in Form einer wässrigen, hydroalkoholischen oder öligen Lösung, oder in Form einer Öl-in-Wasser, Wasser-in-Öl Emulsion, oder multiplen Emulsionen, oder in Form von Cremen, Suspensionen, oder auch Pulver vorliegt; wobei diese Zusammensetzungen können mehr oder weniger flüssig oder fest sein und die Erscheinungsform einer Creme, einer Lotion, einer Milch, eines Shampoos, eines Serums, einer Pomade, eines Gels, einer Paste, eines Schaums oder eines Sticks haben.

16. Kosmetisches Behandlungsverfahren zur Pflege von Keratinsubstraten, insbesondere von Haut und Haaren, umfassend das Aufbringen einer wirksame Menge einer Zusammensetzung wie sie in einem der Ansprüche 11 bis 15 definiert ist, auf Keratinsubstraten, insbesondere auf Haut und/oder Haaren.

17. Kosmetisches Behandlungsverfahren nach Anspruch 16, um die Keratinsubstrate zu schützen, und insbesondere um die Haut und die Haare gegen jegliche Art äußeren Angriffen zu schützen.

18. Kosmetisches Behandlungsverfahren nach Anspruch 16, um die Hautschranke zu verstärken und/oder den Schutz der Haare zu verstärken, und/oder die Synthese von Keratin zu erhöhen und/oder die Keratinsubstrate und insbesondere die Haut und die Haare zu nähren.
